(19) 

**Europäisches Patentamt**
**European Patent Office**
**Office européen des brevets**

(11) **EP 3 374 024 B1**

(12) **EUROPEAN PATENT SPECIFICATION**

(45) Date of publication and mention
of the grant of the patent:
**13.07.2022 Bulletin 2022/28**

(21) Application number: **16863657.9**

(22) Date of filing: **10.11.2016**

(51) International Patent Classification (IPC):
**A61N 7/00** *(2006.01)*    **A61N 7/02** *(2006.01)*
**A61K 38/16** *(2006.01)*    **A61K 38/17** *(2006.01)*
**A61K 49/22** *(2006.01)*    **A61K 49/18** *(2006.01)*
**A61B 18/00** *(2006.01)*    **A61P 35/00** *(2006.01)*
**A61K 51/10** *(2006.01)*    **C07K 16/22** *(2006.01)*
**A61K 39/395** *(2006.01)*    **A61B 90/00** *(2016.01)*
**A61K 39/00** *(2006.01)*

(52) Cooperative Patent Classification (CPC):
(C-Sets available)
**A61K 51/1045; A61B 90/37; A61K 39/395;**
**A61N 7/02; A61P 35/00; A61P 43/00; C07K 16/22;**
A61K 2039/505; A61K 2039/545; A61N 2007/0021;
C07K 2317/24                                    (Cont.)

(86) International application number:
**PCT/CN2016/105293**

(87) International publication number:
**WO 2017/080481 (18.05.2017 Gazette 2017/20)**

(54) **METHOD AND KIT FOR TREATING BRAIN TUMOR BY USING ULTRASOUND SYSTEM**

VERFAHREN UND KIT ZUR BEHANDLUNG VON HIRNTUMOREN MITTELS
ULTRASCHALLSYSTEM

PROCÉDÉ ET TROUSSE POUR LE TRAITEMENT D'UNE TUMEUR CÉRÉBRALE AU MOYEN D'UN
SYSTÈME À ULTRASONS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **11.11.2015 US 201562253805 P**

(43) Date of publication of application:
**19.09.2018 Bulletin 2018/38**

(73) Proprietor: **Navifus Co., Ltd.**
**Taipei City 104 (TW)**

(72) Inventors:
• **LIU, Hao-Li**
  **Taoyuan City 333 (TW)**
• **CHU, Po-Chun**
  **Taipei City 111 (TW)**
• **CHANG, Ting-Kuang**
  **Taipei City 10553 (TW)**

• **WEI, Kuo-Chen**
  **Taoyuan (TW)**
• **CHEN, Pin-Yuan**
  **Taoyuan (TW)**
• **HUANG, Chiung-Yin**
  **Taoyuan (TW)**

(74) Representative: **Lucke, Andreas**
**Boehmert & Boehmert**
**Anwaltspartnerschaft mbB**
**Pettenkoferstrasse 22**
**80336 München (DE)**

(56) References cited:
**WO-A1-2012/019146    CN-A- 103 002 950**
**US-A1- 2010 010 394**

- ZHANG HONG ET AL: "Ultrasound-mediated transscleral delivery of Avastin to the posterior segment of rabbit eye in vivo", INVESTIGATIVE OPHTHALMOLOGY & VISUAL SCIENCE, ANNUAL MEETING OF THE ASSOCIATION-FOR-RESEARCH-IN-VISION-AND-OPHTHALMOLOGY (ARVO); BALTIMORE, MD, USA; MAY 07 -11, 2017, vol. 55, no. 13, 31 March 2014 (2014-03-31), page 1804, XP009512956, ISSN: 0146-0404
- ANNA G SORACE ET AL: "Molecular ultrasound imaging of tumor response to bevacizumab using a breast cancer animal model", ULTRASONICS SYMPOSIUM (IUS), 2012 IEEE INTERNATIONAL, IEEE, 7 October 2012 (2012-10-07), pages 2306-2308, XP032434625, ISSN: 1948-5719, DOI: 10.1109/ULTSYM.2012.0576 ISBN: 978-1-4673-4561-3
- THAKER ASHESH A ET AL: "Combination therapy of radiofrequency ablation and bevacizumab monitored with power Doppler ultrasound in a murine model of hepatocellular carcinoma", INTERNATIONAL JOURNAL OF HYPERTHERMIA,, vol. 28, no. 8, 1 January 2012 (2012-01-01), pages 766-775, XP009512954, ISSN: 1464-5157, DOI: 10.3109/02656736.2012.724517
- WATANABE R ET AL: "0322: Contrast-Enhanced Ultrasound (CEUS) with Sonazoid in the Assessment of Anti-Angiogenic Effects of Bevacizumab in a Mouse Xenografted Model", ULTRASOUND IN MEDICINE AND BIOLOGY, NEW YORK, NY, US, vol. 35, no. 8, 1 August 2009 (2009-08-01), page S42, XP026377538, ISSN: 0301-5629 [retrieved on 2009-07-24]
- BONNIN PHILIPPE ET AL: "Ultrasound assessment of short-term ocular vascular effects of intravitreal injection of bevacizumab (Avastin( ) ) in neovascular age-related macular degeneration", ACTA OPHTHAMOLOGICA (ONLINE), WILEY-BLACKWELL MUNKSGAARD, DENMARK, UNITED KINGDOM, UNITED STATES, NETHERLANDS, vol. 88, no. 6, 31 August 2010 (2010-08-31), pages 641-645, XP009512953, ISSN: 1755-3768, DOI: 10.1111/J.1755-3768.2009.01526.X
- HOYT K ET AL: "Assessing breast cancer response to bevacizumab using contrast-enhanced ultrasound: Initial results using a murine model", ULTRASONICS SYMPOSIUM (IUS), 2009 IEEE INTERNATIONAL, IEEE, PISCATAWAY, NJ, USA, 20 September 2009 (2009-09-20), pages 259-262, XP031654501, ISBN: 978-1-4244-4389-5
- HOYT KENNETH ET AL: "Determination of breast cancer response to bevacizumab therapy using contrast-enhanced ultrasound and artificial neural networks", JOURNAL OF ULTRASOUND IN MEDICINE : OFFICIAL JOURNAL OF THE AMERICAN INSTITUTE OF ULTRASOUND IN MEDICINE ENGLAND APR 2010, JOURNAL OF ULTRASOUND IN MEDICINE : OFFICIAL JOURNAL OF THE AMERICAN INSTITUTE OF ULTRASOUND IN MEDICINE ENGLAND APR 2010, JOUR, vol. 29, no. 4, 31 March 2010 (2010-03-31) , pages 577-585, XP009512955, ISSN: 1550-9613, DOI: 10.7863/JUM.2010.29.4.577
- RICHARD T FRANK ET AL: "Strategies for enhancing antibody delivery to the brain", BBA - REVIEWS ON CANCER, ELSEVIER SCIENCE BV, AMSTERDAM, NL, vol. 1816, no. 2, 3 July 2011 (2011-07-03) , pages 191-198, XP028392590, ISSN: 0304-419X, DOI: 10.1016/J.BBCAN.2011.07.002 [retrieved on 2011-07-08]
- HENDRICKS BENJAMIN K ET AL: "Novel delivery methods bypassing the blood-brain and blood-tumor barriers", NEUROSURGICAL FOCUS, AMERICAN ASSOCIATION OF NEUROLOGICAL SURGEONS, UNITED STATES, vol. 38, no. 3, 28 February 2015 (2015-02-28), pages E10-1, XP009512952, ISSN: 1092-0684, DOI: 10.3171/2015.1.FOCUS14767
- RODRIGUEZ A ET AL: "Neurosurgical techniques for disruption of the blood brain barrier for glioblastoma treatment", PHARMACEUTICS,, vol. 7, no. 3, 3 August 2015 (2015-08-03), pages 175-187, XP009512950, ISSN: 1999-4923, DOI: 10.3390/PHARMACEUTICS7030175
- LIU HAO-LI ET AL: "Blood-brain barrier disruption with focused ultrasound enhances delivery of chemotherapeutic drugs for glioblastoma treatment.", RADIOLOGY MAY 2010, vol. 255, no. 2, May 2010 (2010-05), pages 415-425, XP002791060, ISSN: 1527-1315
- GIL-GIL MIGUEL J ET AL: "Bevacizumab for the treatment of glioblastoma.", CLINICAL MEDICINE INSIGHTS. ONCOLOGY 2013, vol. 7, 2013, pages 123-135, XP002791061, ISSN: 1179-5549
- Anonymous: "Mechanical index", Wikipedia , 7 May 2019 (2019-05-07), XP002791062, Retrieved from the Internet: URL:https://en.wikipedia.org/wiki/Mechanical_index [retrieved on 2019-05-07]
- LIU, H. L. et al.: "Focused Ultrasound Enhances Central Nervous System Delivery of Bevacizumab for Malignant Glioma Treatment.", Radiology., vol. 281, no. 1, 1 October 2016 (2016-10-01), pages 99-108, XP055541882, DOI: 10.1148/radiol.2016152444

- **BHOWMIK, A. et al.: "Blood Brain Barrier: A Challenge for Effectual Therapy of Brain Tumors.", BioMed Research International., vol. 2015, 19 March 2015 (2015-03-19), pages 1-20, XP055383981,**
- **RIINA, H. A. et al.: "Superselective intraarterial cerebral infusion of bevacizumab: a revival of interventional neuro-oncology for malignant glioma.", Journal of Experimental Therapeutics and Oncology., vol. 8, no. 2, 12 September 2009 (2009-09-12), pages 145-150, XP055383984,**
- **DENG, C. X.: "Targeted drug delivery across the blood-brain barrier using ultrasound technique.", Ther Deliv., vol. 1, no. 6, 1 December 2010 (2010-12-01), pages 819-848, XP055384046,**

(52) Cooperative Patent Classification (CPC): (Cont.)

C-Sets
**A61K 39/395, A61K 2300/00**

## Description

Glioblastoma multiforme (GBM) patients usually only have a 5-year survival rate under 5% and median overall survival of only 15 months after aggressive surgery and following adjuvant chemotherapy and radiation. Anti-angiogenic therapy is the focus of recent therapeutic development efforts because GBMs are highly vascularized tumors with irregular, extensive vascular proliferation, increased expression of angiogenic factors, and profoundly high levels of secreted vascular endothelial growth factor (VEGF). High GBM-associated VEGF expression results in growth and proliferation of endothelial cells, which correlates with tumor hypoxia and necrosis, and triggers tumor angiogenesis and progression.

Bevacizumab is a humanized monoclonal antibody specifically binds to the VEGF-A isoform and neutralizes endothelial cell proliferation. Bevacizumab reduces tumor neovascularization, improves blood vessel integrity, decreases tumor-associated edema, and improves clinical quality of life. Theoretically, consistent anti-angiogenesis drug administration should lead to vascular network destruction, impeding oxygen and nutrient transport, and ultimately causing tumor starvation. However, these anti-tumor effects are also impeded by "vascular normalization" mediated by the ability of anti-VEGF agents to effectively reduce vascular permeability and temporally reverse abnormal capillary leakage. The transient "vascular normalization" restores BBB integrity and restricts further bevacizumab penetration into brain parenchyma, thereby reducing angiogenic suppression of GBM cells and sustained tumor starvation to improve patient survival.

RICHARD T FRANK ET AL, "Strategies for enhancing antibody delivery to the brain", BBA - REVIEWS ON CANCER, ELSEVIER SCIENCE BV, AMSTERDAM, NL, vol. 1816, no. 2, doi:10.1016/J.BBCAN.2011.07.002, ISSN 0304-419X, (20110703), pages 191 - 198, (20110708) is a review showing the multiple routes for researching possible options to improve antibody transition over the Blood Brain Barrier (BBB).

HENDRICKS BENJAMIN K ET AL, NEUROSURGICAL FOCUS, AMERICAN ASSOCIATION OF NEUROLOGICAL SURGEONS, UNITED STATES, (20150228), vol. 38, no. 3, doi:10.3171/2015.1.FOCUS14767, reviews drug delivery across the BBB.

RODRIGUEZ A ET AL, PHARMACEUTICS,, (20150803), vol. 7, no. 3, doi:10.3390/PHARMACEUTICS7030175, ISSN 1999-4923, pages 175 - 187 mentions bevacizumab and its involvement in BBB transport.

IU HAO-LIET AL, RADIOLOGY MAY 2010, (201005), vol. 255, no. 2, ISSN 1527-1315, pages 415 - 425 is a document showing the application of ultrasound for enhancing BBB transition of BCNU.

## SUMMARY

In light of the foregoing, one of the objectives of the present invention is to provide a compound for use in a method for treating brain cancer by using an anti-angiogenic therapy. The compound for use in a method is able to provide better efficacy while using less dosage of anti-angiogenic medicine.

In order to achieve the aforesaid objectives, the present invention provides Bevacizumab for use in a treatment of a brain tumor, the treatment comprising the following steps:

administering Bevacizumab to a subject; wherein said Bevacizumab is of an amount of 0.011 to 11 mg/kg body weight for a human;
administering an ultrasound-response medium to said subject; and
administering said subject with an ultrasound exposure of 0.47 to 1.26 mechanical index (MI),
wherein said ultrasound-response medium is a plurality of particles; and wherein said ultrasound exposure is administered on a central nervous system of said subject..

Preferably, said Bevacizumab is of an amount of 0.11 to 11 mg/kg body weight. More preferably, said Bevacizumab is of an amount of 1.1 to 11 mg/kg body weight. Preferably, said administering for said Bevacizumab is via intravenous injection.

Preferably, said administering for said ultrasound-response medium is via intravenous injection. Preferably, said ultrasound-response medium is a plurality of particles. Preferably, said particles have a mean diameter of 0.1 to 10 $\mu$m. Preferably, said plurality of particles is of an amount of $1.9 \times 10^6$ to $1.17 \times 10^8$ particles/kg body weight for said administering. Preferably, said particles are microbubbles.

Preferably, said ultrasound exposure is administered on the central nervous system of said subject. Preferably, said brain tumor is Glioblastoma multiforme.

The present invention also provides a combination of a Bevacizumab formulation and an ultrasound response medium for use in a treatment of a brain tumor, wherein said combination is comprised in a kit, and said kit further comprises an ultrasound system comprising an ultrasound transducer;

wherein an administration of Bevacizumab is in an amount of 0.011 to 11 mg/kg body weight for a human, and said ultrasound system is set to generate an ultrasound exposure of 0.47 to 1.26 MI,
wherein said ultrasound-response medium is a plurality of particles; and wherein said ultrasound exposure is administered on a central nervous system of said subject.

Preferably, said Bevacizumab formulation comprises 0.11 to 25 mg/ml of Bevacizumab and a carrier for injection; wherein said mg/ml is based on the total volume of said Bevacizumab formulation. Preferably, said carrier for injection is water, saline, polymer, emulsifier, surfactant or a combination thereof.
Preferably, said ultrasound-response medium comprises $1 \times 10^4$ to $1 \times 10^{12}$ particle/ml of particles; wherein said particle/ml is based on the total volume of said ultrasound-response medium. Preferably, said particles have a mean diameter of 0.1 to 10 $\mu$m.
Preferably, said ultrasound-response medium is mixed with a carrier for injection. Preferably, said carrier for injection is water, saline, polymer, emulsifier, surfactant or a combination thereof.
Preferably, said brain tumor is Glioblastoma multiforme.
Preferably, said ultrasound system is a medical imaging based guidance ultrasound system. Preferably, said medical imaging comprises neuronavigation, ultrasonography, optical imaging, computed tomography, nuclear imaging (CT), or magnetic resonance imaging (MRI).

## BRIEF DESCRIPTION OF THE DRAWINGS

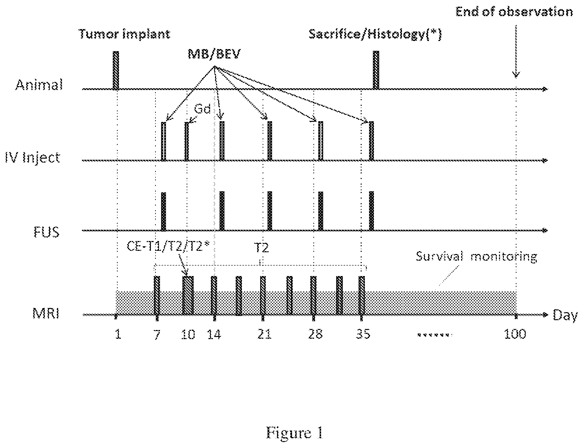

Figure 1 shows the time course for experimental design in using ultrasound-induced BBB opening to enhance Bevacizumab delivery in glioma-bearing mice. Bevacizumab was given by IV at 50 mg/kg body weight weekly (days 1, 8, 15, 22, and 29 after 1st MRI screening), and tumor progression and survival were longitudinally followed by T2-MRI. Ultrasound exposure was conducted weekly (days 1, 8, 15, 22, and 29 after 1st MRI screening, before IV administration of Bevacizumab).

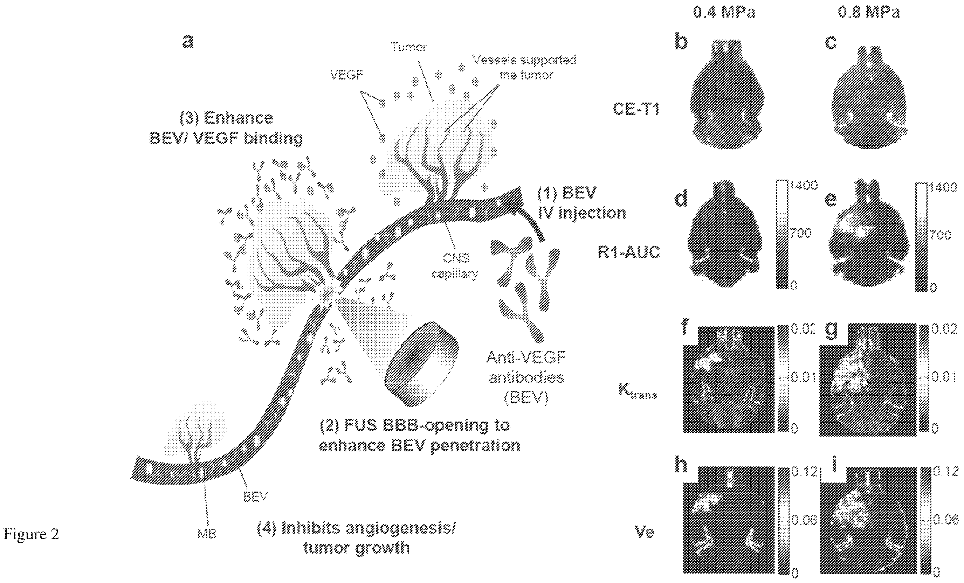

Figure 2 illustrates the concepts and representative MRI observations of ultrasound induced blood-brain barrier (BBB) opening. (a) Conceptual diagram for experimental approach using ultrasound-induced blood-brain barrier opening to enhance Bevacizumab delivery for glioma treatment. (B to I) Representative CE-MRI analysis of ultrasound-induced BBB opening with intermediate (0.4 MPa, i.e. 0.63 MI) and aggressive (0.8 MPa, i.e. 2 MI) ultrasound exposure level. (b, c) Signal intensity level increase maps of contrast-enhanced T1-weighted images (in %); (d, e) area-under-the-curve (AUC) accumulation of R1 maps (in $s^{-1}$.min); (f, g) Ktrans maps (in $min^{-1}$); (h, i) Ve maps.

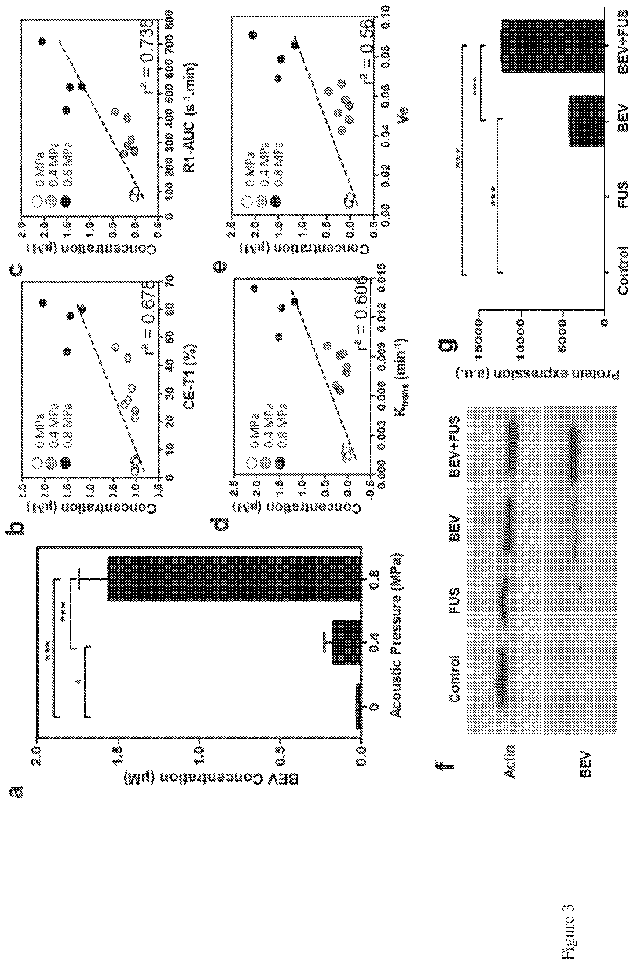

Figure 3 shows the quantitation of delivered Bevacizumab penetration, correlation with MR imaging, and Western blotting. (a) Bevacizumab concentration with different ultrasound exposures (0, 0.4, and 0.8 MPa; i.e. 0, 0.63, and 2 MI respectively) level measured by HPLC; (b-e) Correlations of the Bevacizumab concentration with different indexes of contrast-enhanced MRI including SI increase in T1-WI (penal (b)), R1-AUC level change (penal (c)), Ktrans level change (penal (d)), and Ve level change (penal (e)). (f) Western blot showing enhanced Bevacizumab penetration into CNS after ultrasound-induced BBB opening. (g) Comparison of quantitative analysis of the Western blot intensity. The symbol "***" represents $p < 0.001$. BEV = Bevacizumab.

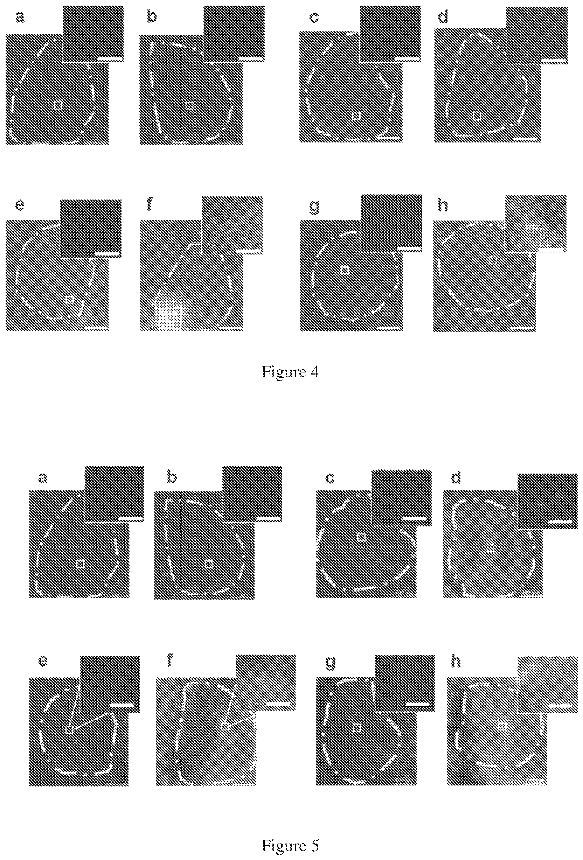

Figure 4 displays the fluorescent microscopic observations of ultrasound-assisted 70-kDa fluorescent-tagged dextrans delivery into CNS. (a, b) Left and right hemispheres of the controlled animal without ultrasound exposure and no fluorescent signal been detected due to the intact BBB. (c, d) animal treated with 0.3-MPa ultrasound exposure at the right hemisphere (fluorescent intensity of 42354$\pm$3937 a.u.). (e, f) animal treated with 0.4-MPa ultrasound exposure (i.e. 0.63 MI) at the right hemisphere (fluorescent intensity of 81329$\pm$7926 a.u.). (g, h) animal treated with 0.53 MPa ultrasound exposure (i.e. 0.84 MI) at the right hemisphere (fluorescent intensity of 85348$\pm$1050 a.u.). Bar = 100 $\mu$m.

Figure 5 displays the fluorescent microscopic observations of ultrasound-assisted 150-kDa fluorescent-tagged dextrans delivery into CNS. (a, b) Left and right hemispheres of the controlled animal without ultrasound exposure and no fluorescent signal been detected due to the intact BBB. (c, d) animal treated with 0.3 MPa/0.4-MHz ultrasound exposure at the right hemisphere (fluorescent intensity of 22716$\pm$3706 a.u.). (e, f) animal treated with 0.4-MPa ultrasound exposure (i.e. 0.63 MI) at the right hemisphere (fluorescent intensity of 29208$\pm$7518 a.u.). (g, h) animal treated with 0.53 MPa/0.4-MHz ultrasound exposure (i.e. 0.84 MI) at the right hemisphere (fluorescent intensity of 52643$\pm$11644 a.u.). Bar = 100 $\mu$m.

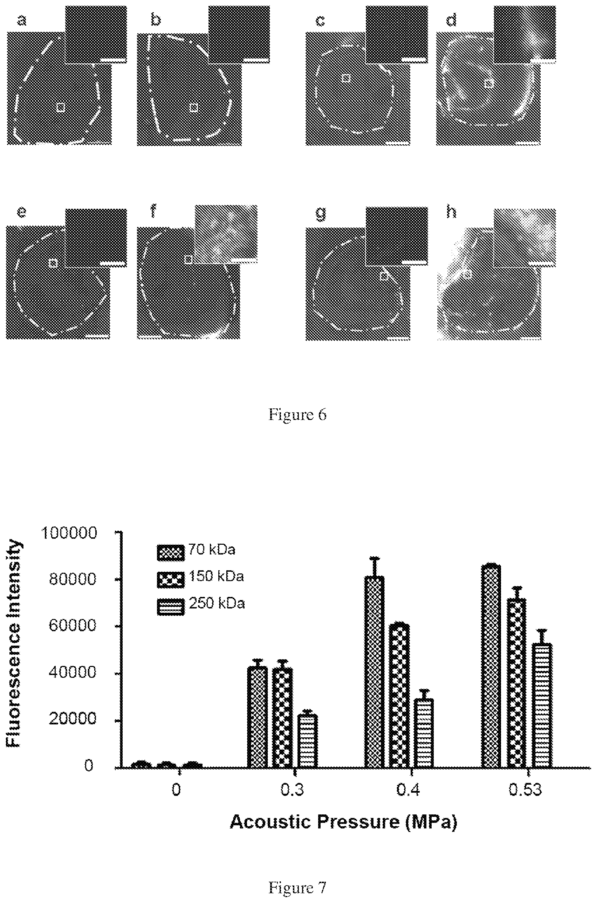

Figure 6 displays the fluorescent microscopic observations of ultrasound-assisted 250-kDa fluorescent-tagged dextrans delivery into CNS. (a, b) Left and right hemispheres of the controlled animal without ultrasound exposure and no fluorescent signal been detected due to the intact BBB. (c, d) animal treated with 0.3 MPa (i.e. 0.47 MI) ultrasound exposure at the right hemisphere (fluorescent intensity of $42012 \pm 5764$ a.u.). (e, f) animal treated with 0.4 MPa ultrasound exposure (i.e. 0.63 MI) at the right hemisphere (fluorescent intensity of $60504.81 \pm 1956.97$ a.u.). (g, h) animal treated with 0.53 MPa (i.e. 0.84 MI) ultrasound exposure at the right hemisphere (fluorescent intensity of $71672 \pm 7770$ a.u.). Bar = 100 $\mu$m.

Figure 7 shows the comparison of the fluorescence intensity change under various triggered pressure and molecular sizes enhanced by ultrasound. Control animal without receiving ultrasound exposure do not allow dextrans penetration due to the blockage of BBB. Higher fluorescence intensity can be detected when higher acoustic pressure was applied or smaller size of fluorescent-tagged dextrans been administered.

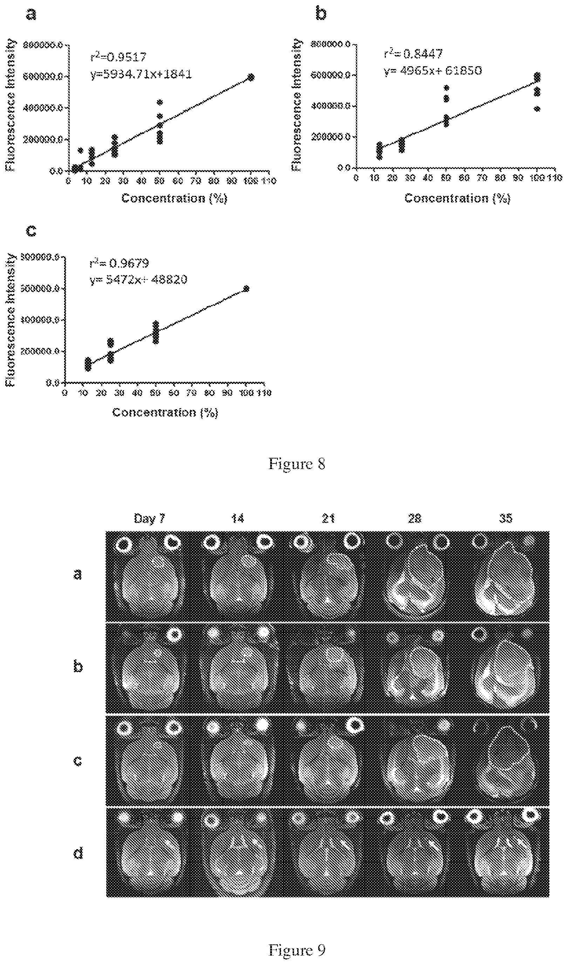

Figure 8 indicates the correlations of the fluorescence intensity with the concentrations of three fluorescent-tagged dextrans. (a-c) Various size of fluorescent-tagged dextrans all show high correlation between the fluorescence intensity to the concentration ($r2 = 0.9517$, $0.8447$, and $0.9679$ for 70-kDa (penal (a)), 150 -kDa (penal (b)), and 250 k-Da (penal (c)), respectively).

Figure 9 displays the representative T2-weighted MRI to follow tumor progression. (a-d) T2-weighted MRI was employed to monitor brain tumor progression by weekly from days 7 to 35 in each experimental group. Ultrasound-induced BBB opening alone (penal (b)) presented similar tumor progression to the untreated control (penal (a)), whereas bevacizumab administration alone provided improved tumor progression suppressed (penal (c)). Among groups, combined ultrasound-induced BBB opening with bevacizumab administration provided the most significant tumor-suppressing effect (penal (d)).

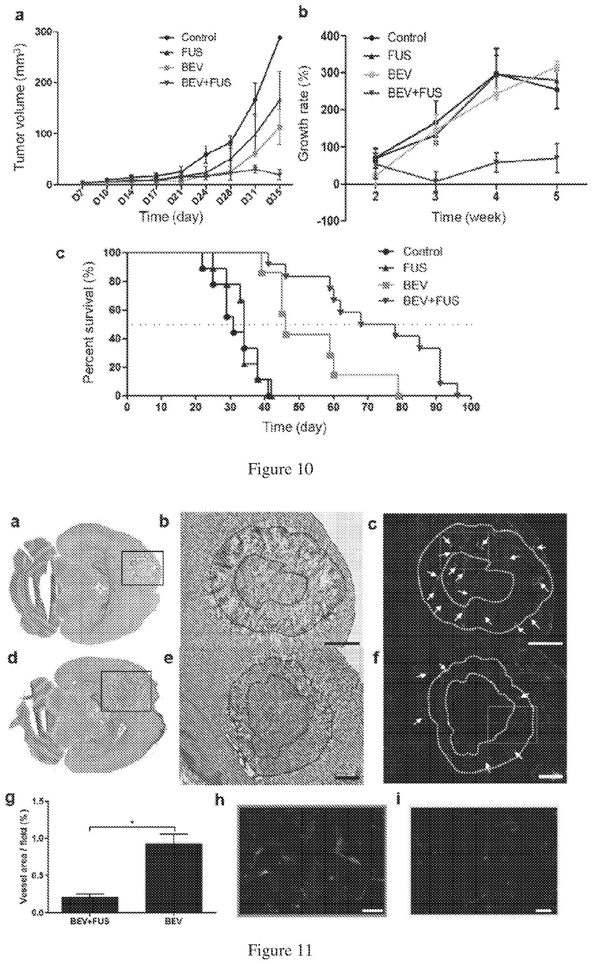

Figure 10 shows the tumor progression and animal survival analysis. (a, b) Tumor progression from days 7 to 35 in each experimental groups. Penal (a) shows tumor volume (in mm3) and penal (b) shows tumor weekly progression rate (in %). (c) Kaplan-Meier plot to demonstrate animal survival among each experimental groups. BEV = Bevacizumab.

Figure 11 shows the comparison of HE-stains and CD-31-stained immunochemistry (IHC) fluorescent microscopy. (a-c) HE-stain and CD-31 IHC microscopy show brain tumor treated with bevacizumab administration alone. (d-f) HE-stain and CD-31 IHC microscopy show brain tumor treated with combined ultrasound-induced BBB opening with bevacizumab administration. Bar = 500 $\mu$m. (g) Quantitative comparison of the vessel density measured within the tumor regions. (h, i) Zoomed images show CD-31 labeled vascularature obtained from (C) and (F), respectively. Bar = 100$\mu$m.

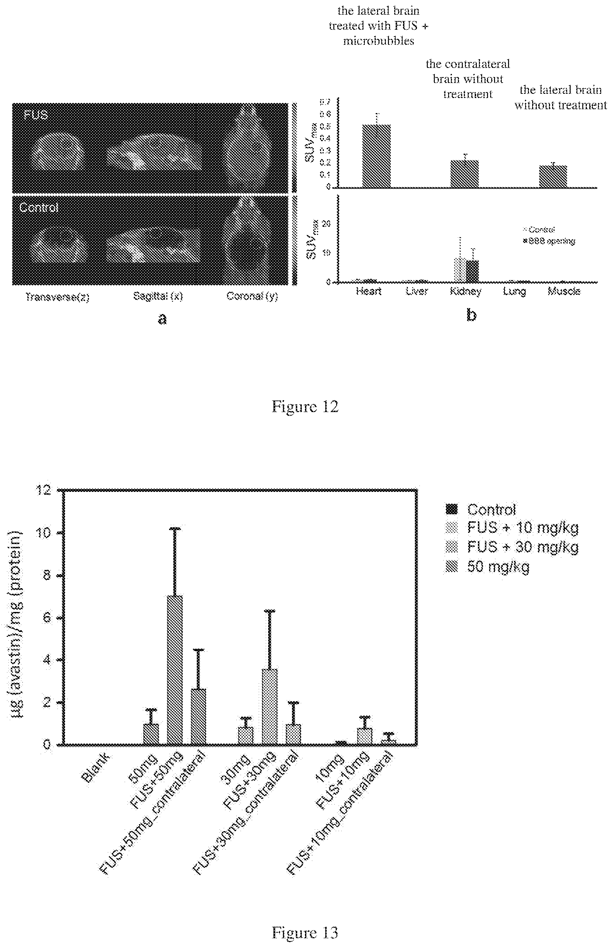

Figure 12 shows the represented decay-corrected PET/CT images of BBB-opening mice at 15 min post-injection of $^{68}$Ga-bevacizumab. a. Experimental group (n=3), mice were treated with ultrasound and injected with 68Ga-bevacizumab. Circles indicate the ultrasound targeting sites. Control group (n=3) with the same dose injection of $^{68}$Ga-bevacizumab, but without the ultrasound treatment. b. PET quantification of BBB opening effect, and biodistribution profile of major organs (Heart, Liver, kidney, Lung, Muscle) among both groups.

Figure 13 shows the quantitation of delivered Bevacizumab penetration. "Blank" represents the control group (untreated group). "50 mg", "30 mg" and "10 mg" represent the groups treated with 50 mg/kg body weight, 30 mg/kg body weight and 10 mg/kg body weight of bevacizumab respectively without ultrasound exposure. "Ultrasound+50 mg", "ultrasound+30 mg" and "ultrasound+10 mg" represent the groups treated with 50 mg/kg body weight, 30 mg/kg body weight and 10 mg/kg body weight of bevacizumab respectively with ultrasound exposure.

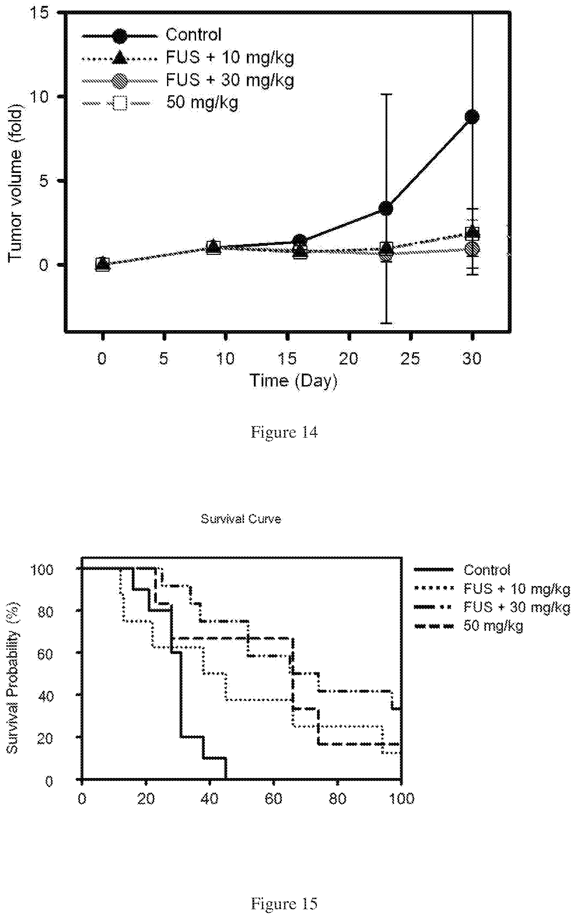

Figure 14 shows the progress of tumor by volume. "Control" represents the control group (untreated group). "50 mg" represents the group treated with 50 mg/kg body weight of bevacizumab without ultrasound exposure. "ultrasound+30 mg" and "ultrasound+10 mg" respectively represent the groups treated with 30 mg/kg body weight or 10 mg/kg body weight of bevacizumab with ultrasound exposure.

Figure 15 demonstrates the survival probability of mice model experiment. "Control" represents the control group (untreated group). "50 mg" represents the group treated with 50 mg/kg body weight of bevacizumab without ultrasound exposure. "ultrasound+30 mg" and "ultrasound+10 mg" respectively represent the groups treated with 30 mg/kg body weight or 10 mg/kg body weight of bevacizumab with ultrasound exposure.

## DETAILED DESCRIPTION

Low-pressure burst-mode transcranial ultrasound exposure can locally, temporally and reversibly open the BBB. Transcranial ultrasound exposure is capable of noninvasive delivery of focal energy to deep-seated brain locations. BBB disruption can increase the local concentration of therapeutic agents in the brain without damaging normal tissue.

The term "effective amount" used herein is referred to an amount of reagent, drug or medicine that is sufficient to obtain the desired effect for the subject in need. The description of "treating brain tumor" or alike used herein is referred to but is not limited to reduce and/or prevent from the progress of the tumor, to reduce the size of the tumor, to reduce and/or prevent from the effect of the tumor on the normal physiology of a subject, or a combination thereof.

The term "ultrasound-response medium" used herewith is referred to as a medium which is able to provide cavitation in response to the acoustic power of ultrasound. In a preferable embodiment, said cavitation is able to open the BBB and more preferably to increase the permeability across the BBB. Without being bound by theory, said ultrasound-response medium is used synergically with said ultrasound exposure to open or disrupt the BBB and thereby increasing the permeability of medicine.

One aspect of the present invention is to provide Bevacizumab for use in a treatment of a brain tumor according to the appended claims. By using the Bevacizumab for use of the invention, the effective amount of Bevacizumab required for treating brain tumor can be reduced. In an alternative embodiment, said brain tumor could be meningiomas or astrocytomas. In a specific embodiment, said astrocytomas is Glioblastoma multiforme.

The Bevacizumab for use of the present invention comprises administering Bevacizumab to a subject; administering an ultrasound-response medium to said subject; and administering said subject with an ultrasound exposure. In an alternative embodiment, there is no particular order for conducting the three steps above.

In an embodiment, said Bevacizumab is of an amount of of 0.11 to 11 mg/kg body weight; more preferably, is 1.1 to 11 mg/kg body weight.

In a preferable embodiment, an amount of $1.9 \times 10^6$ to $1.17 \times 10^8$ particles/kg body weight of said particles are administered. In an alternative embodiment, said particles have a mean diameter of 0.1 to 10 $\mu$m.

Preferably, said particles are microbubbles. In an alternative embodiment, said microbubble has a core-shell structure; wherein said shell is made of biocompatible materials (including but not limited to albumin, lipid, or polymer alike) and the core is a biocompatible gaseous medium. In an alternative embodiment, said microbubble comprises albumin-coated microbubble, lipid-shelled microbubble, gas-filled microbubble, or a combination thereof. In another alternative embodiment, said microbubble may be commercially available product, such as products of SonoVue®, Definity®, Optison®, Imagent®, Levovist®, or Lumason®.

In an alternative embodiment, said ultrasound exposure is of 0.55 to 0.84 MI (Mechanical index; defined as

$$P/\sqrt{f}$$

, where P is peak negative pressure (in MPa) and f is frequency (in MHz)). Preferably, said ultrasound exposure is conducted on the central nervous system of the subject; more preferably, on the brain of the subject.

Another aspect of the present invention is a combination of a Bevacizumab formulation and an ultrasound response medium for use in a treatment of a brain tumor wherein said combination is comprised in a kit, and said kit further comprises an ultrasound system comprising an ultrasound transducer according to the appended claims. In an alternative embodiment, said brain tumor could be meningiomas or astrocytomas. In a specific embodiment, said astrocytomas is Glioblastoma multiforme. In an alternative embodiment, said Bevacizumab formulation comprises 0.11 to 25 mg/ml of Bevacizumab and a carrier for injection. Said carrier for injection could be but not limited to water, saline, polymer, emulsifier, surfactant or a combination thereof.

Said ultrasound-response medium of the kit of the present invention is the same as set forth in the preceding paragraphs regarding the method of the present invention. In a preferable embodiment, said ultrasound-response medium is mixed with a carrier for injection as a formulation and said formulation comprises $1 \times 10^4$ to $1 \times 10^{12}$ particle/ml of particles; wherein said particle/ml is based on the total volume of said formulation. Alternatively, said carrier for injection is water, saline, polymers, emulsifier, surfactant or a combination thereof. In a preferably embodiment, said particles are formulated as a preparation comprising said particles, saline, and heparin.

A typical ultrasound system usually includes an ultrasound transducer and a water tank. In a specific embodiment, said ultrasound system is a medical imaging based guidance ultrasound system. In a preferable embodiment, said ultrasound system is a neuronavigation-guided ultrasound system, which further includes a neuronavigation system (Tsai et al, Ultrasonics Symposium (IUS), 2015 IEEE International). In an alternative embodiment, said ultrasound system could be an ultrasonography-guided ultrasound system, an optical imaging-guided ultrasound system, a computed tomography-guided ultrasound system, a nuclear imaging (CT) - guided ultrasound system, or a MRI-guided ultrasound system.

The following experiments and details are provided for further explanation to the spirit and conception of the present invention. It shall be noted that the following information does not intend to limit the claim scope of the present invention. Those having ordinary skill in art would be able to make modification to the following detailed description of the present invention without apart from the spirit of the present invention.

**Experiments 1 to 4.**

**Materials and Methods of the following Experiments 1 to 4**

1. U87 glioma animal model.

U87 mouse glioma cells were cultured at 37°C with 5% $CO_2$ in MEM with 10% fetal bovine serum and 1% penicillin/streptomycin (Invitrogen). Pathogen-free male NU/NU mice (5-7 weeks old, 20-25 gram) from BioLASCO (Taiwan) were housed in a controlled environment and all experiments were approved by our Institutional Animal Care and Use Committee. To implant U87 cells, animals were anesthetized with 2% isoflurane gas and immobilized on a stereotactic frame. A sagittal incision was made through the skin overlying the calvarium, and a 27G needle was used to create a hole in the exposed cranium 1.5 mm anterior and 2 mm lateral to the bregma. Five microliters of U87 cell suspension ($1 \times 10^5$ cell/$\mu$l) was injected at a depth of 2 mm from the brain surface over a 5-minute period, and the needle was withdrawn over 2 minutes. Xenograft growth was monitored by MRI for 10 days post implantation.

2. Experimental Design.

Twelve normal and 42 tumor-bearing mice were used. In group 1, the aim was to assess if ultrasound-induced BBB opening enhanced bevacizumab penetration in normal mice (n = 4), and to determine the appropriate ultrasound power range (n=4) and for Western blot analysis (n = 8; 4 normal and 4 tumor-bearing). In group 2, the aim was to evaluate the therapeutic efficacy of bevacizumab combined with ultrasound exposure in tumor-bearing mice. There were 4 sub-groups: (1) sham (n = 7); (2) ultrasound alone (n = 9); (3) bevacizumab alone (n = 6), bevacizumab was given by IV at 50 mg/kg body weight weekly (days 1, 8, 15, 22, and 29 after 1st MRI screening); and (4) bevacizumab + ultrasound-BBB opening (n = 12), bevacizumab was given by IV at 50 mg/kg body weight weekly (days 1, 8, 15, 22, and 29 after 1st MRI screening). In group 2, the endpoint was MRI-measured tumor volume > 200 $mm^3$ or higher than 20% body weight drop during one week. The time course for the experimental design is shown in **Figure** 1.

3. Ultrasound exposure.

The ultrasound is a neuronavigation-guided ultrasound according to Tsai et al, Ultrasonics Symposium (IUS), 2015 IEEE International. An ultrasound transducer (Sonic Concepts Inc., Washington, USA; diameter = 60 mm, radius of curvature = 52 mm, frequency = 400 kHz) was applied to generate concentrated ultrasound energy. An arbitrary function generator (33220A, Agilent, Palo Alto, CA) was used to produce the driving signal, which was fed to a radio frequency power amplifier (No. 500-009, Advanced Surgical Systems, Tucson, AZ) operating in burst mode. Anesthetized animals were immobilized on a stereotactic frame and a PE-10 catheter was inserted into tail veins. The animal was placed directly under an acrylic water tank with the head firmly attached to a $4 \times 4$ $cm^2$ thin-film window at the bottom to allow entry of ultrasound energy. SonoVue$^\circledR$ $SF_6$-filled ultrasound particles (2-5 $\mu$m, 10 $\mu$l/mouse, $2 \times 10^8$ microbubble/ml; Bracco, Milan, Italy) were administered intravenously before ultrasound. The tumor-implant hemisphere brain site was then exposed to burst-tone mode ultrasound (electrical power = 4-18 W; peak negative pressure = 0.1 to 2 MI; burst length = 10 ms; pulse repetition frequency = 1 Hz; exposure time = 60 s).

4. Magnetic resonance imaging (MRI).

Contrast-enhanced MRI was used to assess increased BBB permeability via Gd-DTPA administration (Magnevist, Wayne, NJ, USA), and obtain the following imaging indexes: (1) signal intensity increase in T1-weighted images after Gd-DTPA injection 10; (2) Area-under-the-curve of the R1 relaxivity over a period (denoted as R1-AUC); (3) the kinetic parameter "Ktrans", transfer rate constant from the intravascular system to the extracellular extravascular space, characterized from Kety's compartment model; (4) the kinetic parameter "Ve", volume fraction of the contrast agent in the EES, characterized from Kety's compartment model.
Susceptibility-weighted imaging sequences were acquired to identify large-scale erythrocyte extravasations (parameters: TR/TE = 30 ms/18 ms; flip angle = 40°; slice thickness = 0.6 mm; matrix size = 256 $\times$ 384; and FOV = 80 $\times$ 130 $mm^2$). MRI images were acquired on a 7-Tesla magnetic resonance scanner (Bruker ClinScan, Germany) and a 4-channel surface coil was used on the top of the mouse brain. Anesthetized animals were placed in an acrylic holder and positioned in the magnet center. Tumor size was quantified using turbo-spin-echo based T2-weighted images with the following parameters: pulse repetition time (TR)/ echo time (TE) = 2540/41 ms; FOV = 19$\times$30 $mm^2$ (156$\times$320 pixels); slice thickness = 0.5 mm; flip angle =180; total acquisition time = 155 seconds. Relative tumor size was estimated by measuring the single image slide containing the maximum tumor area, and animals were longitudinally imaged every 7 days for up to 35 days after the first MRI screen.

5. Histological examination.

Evans Blue dye was injected intravenously and animals were sacrificed after two hours. Histopathology was performed on 10-$\mu$m sections from paraformaldehyde-fixed, paraffin-embedded brains. Slides were placed in a staining jar containing a hydrochloric acid-potassium ferrocyanide solution for 30 minutes at room temperature. The slides were counterstained by nuclear fast red for 5 min. Hematoxylin and eosin (H&E) staining was used to evaluate brain tissue damage and tumor progression.

To assess vascularity, slides were subjected to immunofluorescence with antimouse-CD31 antibody (1:500, 550274, BD Pharmingen, NJ, USA) and goat-antirat Dylight 488 antibody (1:200, 405409, Biolegend, CA, USA). Tumor blood vessels were quantified as total vessel area relative to tumor cross-sectional area.

6. Western blot.

50$\mu$g protein (goat-anti-Human IgG, AP112P, Millipore, USA) from homogenized brain tissues was used for Western blot analysis. Band optic density on film was analyzed using the BioSpectrum Imaging System (UVP LLC, Upland, CA).

7. Quantitative bevacizumab analysis.

Animals were sacrificed 2 hours after ultrasound and brains were divided into left and right hemispheres and homogenized with 10$\mu$l/mg mL methanol. Extracted Bevacizumab was analyzed by HPLC with a Model L-2400 UV detector and Model L-2130 pump (Hitachi), and a SUPELCOSIL™ LC-18 column (4.6 • 250 mm).

8. Statistical analysis.

Statistical significance was calculated using either two-tailed unpaired t test or Mantel-Cox test. The Kaplan-Meier method was used for survival analysis. Statistical significance was assumed at $p < 0.05$.

**Experiment 1: Ultrasound effectively opens BBB and increases the concentration of bevacizumab in the brain.**

In this experiment, transcranial ultrasound exposure of 0.63 MI or 2 MI (i.e. with a negative peak pressure of 0.4 MPa or 0.8 MPa) was performed in accordance with the method set forth above to evaluate its effect on opening the BBB. Also, bevacizumab was administered before the ultrasound exposure was performed to see if the ultrasound exposure contributed any benefit to the penetration of Bevaczumab through the BBB.

The opening of BBB was confirmed by contrast-enhanced MRI. Four types of CE-MRI indexes were used for this confirmation. The result shown in **Figure 2** indicated that both of 0.63 MI and 2 MI ultrasound exposure effectively opened the BBB at target brain regions. Moreover, higher pressure contributed to greater BBB-opening effect.

HPLC was used to quantitate bevacizumab concentration in the brain. The result showed that ultrasound increased bevacizumab concentration in CNS parenchyma. The intermediate 0.63 MI ultrasound exposure resulted in 0.175$\pm$0.15 $\mu$M of bevacizumab penetration into CNS (i.e., 5.73-fold increase), whereas aggressive 2 MI ultrasound exposure significantly increased bevacizumab to 1.554$\pm$0.37 $\mu$M (i.e., 58.77-fold increase) **(Figure 3a)**. In controls, bevacizumab is normally limited to the circulation, and penetrated the CNS in very limited amounts (0.026$\pm$0.02 $\mu$M). The four CE-MRI indices with correlations to bevacizumab concentrations were also analyzed **(Figures 3b-e)**. R1-AUC was observed to have the highest linear correlation with the measured bevacizumab level **(Figure 3c;** $r^2$ = 0.738). Either increased signal intensity in T1-weighted images or Ktrans provided a moderate correlation to bevacizumab level with a correlation coefficient higher than 0.6 ($r^2$ = 0.678 and 0.606, respectively; **Figure 3b and 3d),** whereas Ve was least correlated with the bevacizumab level ($r^2$ = 0.56; **Figure 3e).** Overall, the four indices provided sufficient correlation with the in vivo ultrasound-induced BBB opening enhancement of CNS bevacizumab delivery.

**Experiment 2: The effect of ultrasound-induced BBB opening on molecular penetration of different molecular size.**

To understand the influence of molecular penetration with the molecular size and ultrasound exposure level, fluorescent-tagged dextrans (70 to 250 kDa) with similar molecular weight to bevacizumab (150 kDa) were served as surrogates to fine-tune the exposure level (0.55 to 0.84 MI) around the intermediate exposure level (i.e., 0.4 MPa/0.63 MI). Pressure level of above 0.55 MI were found to able to induce BBB opening and allow penetration of all sizes of molecule, including the 70- kDa **(Figure 4)**, 150-kDa **(Figure 5),** and 250-kDa **(Figure 6)** fluorescent-tagged dextrans, suggesting 0.63 MI exposure level should provide stable and consistent molecular penetration with the molecule at least up to 250 kDa. Yet, the fluorescent intensity increased in the brain either increasing the ultrasound exposure level or decreasing the molecular weight, suggesting smaller molecular or higher exposure level contributes to higher molecular penetration **(Figure 7)**. Ultrasound exposure increased to 0.84 MI began to induce noticeable but minimal extravasations of erythrocytes **(Figure 8c),** but not observed at lower exposure level **(Figures 8a-b).** These extravasations caused by 0.84 MI exposure was unable to be detected in T2* MRI; in contrast, 2 MI exposure induced apparent drop in signal intensity in T2* MRI, suggesting a relatively larger scale of extravasations of erythrocytes been induced than 0.84 MI exposure. Considering together, the 0.63 MI ultrasound exposure level was selected for the following glioma-bearing tumor sonication.

Western blots were performed to measure bevacizumab protein levels in ultrasound-exposed brains **(Figure 3f).** No bevacizumab was detected in either control or ultrasound-alone animals. With bevacizumab administration alone, only a 150 kDa band was detected, indicating the presence of bevacizumab in the CNS. In contrast, the combination of ultrasound exposure (0.63 MI) with bevacizumab administration markedly increased the levels of detectable bevacizumab protein. Ultrasound with bevacizumab administration produced a nearly 3-fold increase in hybridization intensity **(Figure 3g),** which was similar to the improved penetration determined by HPLC analysis (12,238 versus 4,138 in detected counts).

**Experiment 3: Ultrasound and Bevacizumab combination strategy for glioma therapy**

We aimed to evaluate whether the enhanced bevacizumab delivery with ultrasound-induced BBB opening improved glioma therapy. The ultrasound exposure level of 0.63 MI was selected to avoid accompanying erythrocyte extravasation for the planned five-week repetitive treatment regimen. Please see the "Experiment design" section and Figure 1.

Typical tumor follow-up images are shown in **Figure 9** (days 7 to 35), with the tumor volume progression measured by T2-MRI **(Figures 10a-b).** Overall, the untreated controls **(Figure 9a)** and ultrasound-exposure alone **(Figure 9b)** showed progressive tumor growth (tumor progression ratios with follow-up interval of 10-35 days were $172\pm33.2\%$ and $185\pm22.6\%$, respectively). Treatment with bevacizumab alone **(Figure 9b)** showed a tumor suppressive effect in the first four treatment weeks, but tumor progression surged at week 5. The overall tumor progression ($152\pm27.7\%$) was not significantly different from the control (p = 0.6477) or the ultrasound-alone treatment (p = 0.3735) groups. Bevacizumab administration combined with ultrasound-BBB opening **(Figure 9d)** consistently reduced tumor progression throughout the five-week treatment regimen, and showed the most profound control of tumor progression when evaluated via T2-MRI ($45\pm16.11\%$, p=0.0016 vs. control group; **Figures 10a-b).** This result suggests that enhanced bevacizumab penetration facilitated by ultrasound exposure contributes to a stable and sustained anti-angiogenic effect to control tumor progression.

The combined ultrasound and bevacizumab treatment was conducted for 5 weeks (days 7-35), then animal survival at 100 days evaluated **(Figure 10c** and **Table 1).** ultrasound-only animals did not show effective extension of survival, the same as observed in MRI-monitored tumor progression (median survival = 34 days when compared to 31 days in control; ISTmedian = 9.68%; p = 0.5407). Five-week bevacizumab administration contributed to significantly prolonging survival to 46 days compared to controls (ISTmedian = 48.39%; p = 0.0369). The combined ultrasound-induced BBB opening and bevacizumab administration strategy resulted in significantly improved median survival of 73 days, which is 2.35-fold higher than the median survival of control and 1.58-fold higher than bevacizumab alone. The equivalent observation also holds when mean survival and ISTmean were calculated.

**Table 1.** Summary of animal survival analysis. The increase in median survival time (ISTmedian; in %) was found to be 135% in combined ultrasound/ bevacizumab group and outperformed to 48% in bevacizumab-alone group. Similarly, the increase in mean survival time (ISTmean; in %) was found to be 143% in combined ultrasound/ bevacizumab group and outperformed to 76% in bevacizumab-alone group; p-values are all relative to the control group.

| Group (n) | Median survival (days) | IST$_{median}$ (%) | Mean survival (days) | IST$_{mean}$ (%) | *p-value* |
|---|---|---|---|---|---|
| Control (7) | 31 | -- | $29.71\pm5.35$ | -- | -- |
| Ultrasound alone (9) | 34 | 9.68 | $33.67\pm4.82$ | 13.33 | 0.5407 |
| Bevacizumab alone (6) | 46 | 48.39 | $52.33\pm14.8$ | 76.14 | 0.0369 |
| Ultrasound + Bevacizumab (12) | 73 | 135.48 | $72.33\pm18.9$ | 143.45 | 0.0001 |

H&E staining and CD-31 immunohisochemistry (IHC) were used to assess morphological changes and vascular distributions of week-4 tumor xenografts after either administration of bevacizumab alone or combined with ultrasound-induced BBB opening **(Figure 11)**. Similar tumor morphology was detected in bevacizumab-alone or bevacizumab + ultrasound-BBB opening: a necrotic tumor core accompanied by highly proliferative tumor rims. CD-31 IHC visualized an enriched vascular structure within the tumor rims, but not the necrotic core, of tumors treated with bevacizumab-alone (marked by white arrows). In contrast, a significant reduction in the CD-31 marked vascular distribution was observed in bevacizumab + ultrasound treated xenografts, and tumor rims were significantly smaller in dimension than tumors treated with bevacizumab alone (contoured by dashed line; **Figure 11c** and **Figure 11f)**. Quantitative analysis of tumor blood vessels demonstrated that the mean ($\pm$SD) percent vessel area in tumor xenografts of animals treated with bevacizumab alone was $0.93\pm0.19\%$. The vessel area was markedly decreased in xenografts treated with combined ultrasound and bevacizumab, with a vessel area of $0.2 \pm 0.07\%$ **(Figure 11c, Figure 11f, Figure 11g)**. Tissue necrosis was also observed by H&E staining in the area surrounding the tumor **(Figure 11b, Figure 11e)**. These data suggest that addition of ultrasound-BBB opening enhanced bevacizumab penetration and effectively suppressed angiogenesis, particularly in the tumor periphery, resulting in improved tumor control.

### Experiment 4

**Experiment 4: Ultrasound-induced BBB opening increases the penetration of bevacizumab**

We had shown that the ultrasound increased the molecular penetration of different molecular size in previous experiment. It was further proposed that the ultrasound exposure can decrease the required dosage of bevacizumab in treating giloblastoma multiforme. In order to verify our hypothesis, normal mice and bearing mice were treated with bevacizumab of various dosages with or without ultrasound. The bevacizumab concentration in CNS parenchyma was determined by HPLC. Penetration curve (given dosage vs. bevacizumab concentration in CNS parenchyma) was made accordingly. The operations of ultrasound and HPLC were the same as set forth in the previous experiments 1-4.

Specifically in the experiments, 42 normal mice and 42 tumor-bearing mice were used. In group 1, mice were given bevacizumab of 50 mg/kg body weight alone (n=3) or with ultrasound (n=3); in group 2, mice were given bevacizumab of 25 mg/kg body weight alone (n=3) or with ultrasound (n=3); in group 3, mice were given bevacizumab of 10 mg/kg body weight alone (n=3) or with ultrasound (n=3); in group 4, mice were untreated (n=3) as negative controls. The bevacizumab concentration can also be determined via microPET/ micro-CT. The following is the primary result of evaluating the dynamic change of bevacizumab distribution via microPET/ micro-CT.

Dynamic change of bevacizumab distribution was evaluated via microPET/ micro-CT fused imaging with bevacizumab been radiolabeled with radioisotope $^{68}$Ga$^{3+}$ (half-life:68 min; procedures see the supplementary methods). The PET images were acquired 15 minutes after intravenous injection and the representative coronal PET images of both groups, with this time point the contrast of targeting region reached maximal as the nonspecific binding.

The $^{68}$Ga-bevacizumab penetration in BBB opening mice has shown significant enhancement in the insonified region by using ultrasound treatment combined particles administration. The penetration of 68Ga-bevacizumab increased from $0.16\pm0.026$ SUVmax to $0.64 \pm0.09$ SUVmax, **(Figure 12a)** corresponding to a 4-fold enhancement, which was comparable to the HPLC quantitated results (5.73-fold). Systemic uptake shows that bevacizumab majorly absorbed and metabolized from kidney. Animals underwent ultrasound-BBB opening do not change the metabolized route of bevacizumab, although there was a slightly SUVmax level decrease when compared to the control groups ($7.53\pm4.05$ SUVmax v.s. $8.19\pm7.29$ SUVmax; p > 0.05; see **Figure 12b).**

### Experiment 5

**Materials and Methods of the following Experiment 5:**

1. Experimental Design

A total of 45 tumor-bearing mice were used, including normal (n = 19) and tumor-bearing mice (n = 26). Experiments were divided into two groups. In experimental group 1, the primary aim was to assess if ultrasound-induced BBB opening promoted Bevacizumab penetration and deposition in brain tissue (n = 19). The majority of normal animals were divided into two groups, without ultrasound-induced BBB opening (n = 10) and with ultrasound-induced BBB opening (n = 9), and the brain samples with the Bevacizumab concentration were quantified using enzyme-linked immunosorbent assay (ELISA) kit. All animals were sacrificed and the brain samples were preserved 2 hours after Bevacizumab administration.

In experimental group 2, the aim was to evaluate the therapeutic efficacy of Bevacizumab combined with ultrasound in tumor-bearing mice. Bevacizumab uptake was performed 10 days after U87 glioma cell implantation.

Bevacizumab was IV administered five times (days 10, 17, 24, 31 and 38 after U87 glioma cell implantation), and the tumor progression and survival were both longitudinally followed. Animals were divided into 4 sub-groups: (1) sham (without Bevacizumab administration) (n = 10); (2) Bevacizumab 10 mg/kg per week for 5 weeks plus ultrasound-induced BBB opening (n = 8); (3) Bevacizumab 30 mg/kg per week for 5 weeks plus ultrasound-BBB opening (n = 12) and (4) Bevacizumab 50 mg/kg per week for 5 weeks (n = 6).

2. Ultrasound Parameter

The ultrasound is a neuronavigation-guided ultrasound according to Tsai et al, Ultrasonics Symposium (IUS), 2015 IEEE International. An ultrasound transducer (Imasonics, Besancon, France; diameter = 60 mm, radius of curvature = 80 mm, frequency = 400 kHz) was applied to generate concentrated ultrasound energy. An arbitrary function generator (33120A, Agilent, Palo Alto, CA) was used to produce the driving signal, which was fed to a radio frequency power amplifier (No. 500-009, Advanced Surgical Systems, Tucson, AZ) operating in burst mode. Animals were anesthetized with 2% isoflurane and immobilized on a stereotactic frame. The top of the cranium was shaved with clippers, and a PE-10 catheter was inserted into the tail vein. The animal was placed directly under an acrylic water tank (with a window of $4 \times 4$ cm$^2$ at its bottom sealed with a thin film to allow entry of the ultrasound energy) with its head attached tightly to the thin-film window. SonoVue SF$_6$-filled ultrasound particles (2-5 $\mu$m, 10 $\mu$l/mouse, $2 \times 10^8$ microbubble/ml; Bracco, Milan, Italy) were administered intravenously before treatment. The tumor implanted brain site was then exposed to burst-tone mode ultrasound to locally open the BBB (0.63 MI; burst length = 10 ms; pulse repetition frequency = 1 Hz; exposure time = 60 s).

3. Mouse Glioma Model

U87 mice glioma cells were cultured at 37°C in a humidified 5% CO$_2$ atmosphere in minimum essential median (MEM) supplemented with 10% fetal bovine serum and 1% penicillin/streptomycin (Invitrogen). Cells were harvested by trypsinization, washed once with phosphate-buffered saline (PBS), and resuspended (1.6 x 10$^5$ cell/$\mu$l ) in MEM for implantation into the striatum of mouse brains. Pathogen-free male NU/NU mice (5 to 7 weeks old) were purchased from BioLASCO (Taiwan). Mice were housed and maintained in a controlled environment and all procedures were performed in accordance with the experimental animal care guidelines of the Animal Committee of Chang Gung University. To implant U87 tumor cells, we anesthetized animals with 2% isoflurane gas and immobilized them on a stereotactic frame. A sagittal incision was made through the skin overlying the calvarium, and a 23G needle was used to create a hole in the exposed cranium 2 mm anterior and 2 mm lateral to the bregma. Three microliters of U87 glioma cell suspension were injected at a depth of 2 mm from the brain surface. The injection was performed over a 3-minute period, and the needle was withdrawn over another 2 minutes. The growth of the mouse brains was monitored by MRI for 10 days post tumor cell implantation.

4. Quantitative Bevacizumab Analysis

Brain tissues were collected after 2 hours after the administration of Bevacizumab. The brain tissues were homogenized and centrifuged at 5000 rpm for 10 minutes until a clear separation between supernatant fluid and the tissue components was seen. The concentration of Bevacizumab was measured with an enzyme-linked immunosorbent assay (ELISA) kit (My Biosource, Inc. San Diego, CA, USA), The optical density was determined at 450 nm with the absorption spectrophotometer with the background subtraction at 620 nm.

**Experiment 5: Ultrasound increases the penetration of bevacizumab and promote the efficacy of bevacizumab in treating glioblastoma multiforme**

According to the result shown in **Figure 13,** it was noted that, basically, higher dosage of Bevacizumab administered resulted in higher penetration of Bevacizumab in the brain. Furthermore, the treatment of 10 mg/kg body weight of Bevacizumab with ultrasound exhibited comparable concentration of Bevacizumab in the brain sample with the treatment of 50 mg/kg body weight of Bevacizumab without ultrasound exposure. The treatment of 30 mg/kg body weight of Bevacizumab with ultrasound showed much more Bevacizumab penetration than the treatment of 50 mg Bevacizumab without ultrasound exposure. The result demonstrated that, with the assistance of ultrasound, less amount of Bevacizumab was required to achieve comparable concentration in the brain. Moreover, the tumor progression was moderated in the groups with treatment in comparison with the control group. In addition, the groups with ultrasound exposure exhibited results though less amount of Bevacizumab was administered **(Figure 14).**

Further in the survival test **(Figure 15),** among others, the treatment of 30 mg/kg body weight of Bevacizumab with ultrasound contributed the best survival probability. The survival probability of the group treated with 10 mg/kg body weight of Bevacizumab with ultrasound was almost as good as the group treated with 50 mg/kg body weight of Bevacizumab without ultrasound. The result was consistent with the penetration experiment set forth above showing that the ultrasound assisted more penetration of Bevacizumab and thereby provided better chance of survival.

**Conclusion**

In conclusion, the use of ultrasound induced blood-brain barrier opening enhances CNS delivery of the antiangiogenic mAb, bevacizumab. The CNS bevacizumab concentration is enhanced by up to 57 fold **(Figure 2a),** resulting in significant anti-angiogenic effect on tumor periphery, and more importantly, overcome the current vascular normalization challenge of bevacizumab in GBM therapy. Moreover, a more profound therapeutic efficacy improvement was found in this study that combined ultrasound to enhance bevacizumab delivery contributes to 135% of median survival improvement, when compared with the improvement of 48% in bevacizumab administration alone **(Table 1).** This study provides proof-of-principle for development of therapeutic strategies that incorporate ultrasound to enhance CNS delivery of monoclonal antibody (or other large molecules), and suggests the ultrasound/bevacizumab combination as a promising anti-GBM strategy.

**Claims**

1. Bevacizumab for use in a treatment of a brain tumor, the treatment comprising the following steps:

   administering Bevacizumab to a subject; wherein said Bevacizumab is of an amount of 0.011 to 11 mg/kg body weight for a human;
   administering an ultrasound-response medium to said subject; and
   administering said subject with an ultrasound exposure of 0.47 to 1.26 mechanical index (MI),
   wherein said ultrasound-response medium is a plurality of particles; and wherein said ultrasound exposure is administered on a central nervous system of said subject.

2. The Bevacizumab for use of claim 1, wherein said ultrasound exposure is of 0.55 to 0.84 MI.

3. The Bevacizumab for use of claim 1 or 2, wherein said Bevacizumab is of an amount of 0.11 to 11 mg/kg body weight for a human.

4. The Bevacizumab for use of any one of claims 1 to 3, wherein said administering for said Bevacizumab is via intravenous injection.

5. The Bevacizumab for use of any one of claims 1 to 4, wherein said administering for said ultrasound-response medium is via intravenous injection.

6. The Bevacizumab for use of any one of claims 1 to 5, wherein said particles have a mean diameter of 0.1 to 10 $\mu$m.

7. The Bevacizumab for use of any one of claims 1 to 6, wherein said plurality of particles is of an amount of $1.9 \times 10^6$ to $1.17 \times 10^8$ particles/kg body weight for said administering.

8. The Bevacizumab for use of any one of claims 1 to 7, wherein said particles are microbubbles.

9. A combination of a Bevacizumab formulation and an ultrasound response medium for use in a treatment of a brain tumor, wherein said combination is comprised in a kit, and said kit further comprises an ultrasound system comprising an ultrasound transducer;

   wherein an administration of Bevacizumab is in an amount of 0.011 to 11 mg/kg body weight for a human, and said ultrasound system is set to generate an ultrasound exposure of 0.47 to 1.26 MI,
   wherein said ultrasound-response medium is a plurality of particles; and wherein said ultrasound exposure is administered on a central nervous system of said subject.

10. The combination for use of claim 9, wherein said Bevacizumab formulation comprises 0.11 to 25 mg/ml of Bevacizumab and a carrier for injection; wherein said mg/ml is based on the total volume of said Bevacizumab formulation, wherein said carrier for injection is preferably water, saline, polymer, emulsifier, surfactant or a combination thereof.

11. The combination for use of claim 9 or 10, wherein said ultrasound-response medium comprises $1 \times 10^4$ to $1 \times 10^{12}$ particle/ml of particles; wherein said particle/ml is based on the total volume of said ultrasound-response medium, wherein said particles preferably have a mean diameter of 0.1 to 10 $\mu$m.

12. The combination for use of any one of claims 9 to 11, wherein said ultrasound-response medium is mixed with a carrier for injection, wherein said carrier for injection is preferably water, saline, polymer, emulsifier, surfactant or a combination thereof.

13. The Bevacizumab for use of any one of claims 1 to 9, or the combination for use of any one of claims 10 to 12, wherein said brain tumor is Glioblastoma multiforme.

14. The combination for use of any one of claims 9 to 13, wherein said ultrasound system is a medical imaging based guidance ultrasound system.

15. The combination for use of claim 14, wherein said medical imaging comprises neuronavigation, ultrasonography, optical imaging, computed tomography (CT), nuclear imaging, or magnetic resonance imaging (MRI).

**Patentansprüche**

1. Bevacizumab zur Verwendung in einer Behandlung eines Gehirntumors, wobei die Behandlung die folgenden Schritte umfasst:

   Verabreichen von Bevacizumab an ein Subjekt; wobei das Bevacizumab in einer Menge von 0,011 bis 11 mg/kg Körpergewicht für einen Menschen ist;
   Verabreichen eines Ultraschallreaktionsmediums an das Subjekt; und
   Verabreichen einer Ultraschallexposition mit einem Mechanischen Index (MI) von 0,47 bis 1,26 an das Subjekt, wobei das Ultraschallreaktionsmedium mehrere Partikel ist; und wobei die Ultraschallexposition an ein zentrales Nervensystem des Subjekts verabreicht wird.

2. Bevacizumab zur Verwendung nach Anspruch 1, wobei die Ultraschallexposition von 0,55 bis 0,84 MI beträgt.

3. Bevacizumab zur Verwendung nach Anspruch 1 oder 2, wobei das Bevacizumab in einer Menge von 0,11 bis 11 mg/kg Körpergewicht für einen Menschen ist.

4. Bevacizumab zur Verwendung nach einem der Ansprüche 1 bis 3, wobei das Verabreichen des Bevacizumab über eine intravenöse Injektion erfolgt.

5. Bevacizumab zur Verwendung nach einem der Ansprüche 1 bis 4, wobei das Verabreichen des Ultraschallreaktionsmediums über eine intravenöse Injektion erfolgt.

6. Bevacizumab zur Verwendung nach einem der Ansprüche 1 bis 5, wobei die Partikel einen mittleren Durchmesser von 0,1 bis 10 $\mu$m aufweisen.

7. Bevacizumab zur Verwendung nach einem der Ansprüche 1 bis 6, wobei die mehreren Partikel in einer Menge von $1,9 \times 10^6$ bis $1,17 \times 10^8$ Partikel/kg Körpergewicht für das Verabreichen ist.

8. Bevacizumab zur Verwendung nach einem der Ansprüche 1 bis 7, wobei die Partikel Mikrobläschen sind.

9. Kombination aus einer Bevacizumab-Formulierung und einem Ultraschallreaktionsmedium zur Verwendung bei einer Behandlung eines Gehirntumors, wobei die Kombination in einem Kit enthalten ist und das Kit ferner ein Ultraschallsystem umfasst, das einen Ultraschallwandler umfasst; wobei eine Verabreichung von Bevacizumab in einer Menge von 0,011 bis 11 mg/kg Körpergewicht für einen Menschen erfolgt und das Ultraschallsystem eingestellt ist, um eine Ultraschallexposition von 0,47 bis 1,26 MI zu erzeugen, wobei das Ultraschallreaktionsmedium mehrere Partikel ist; und wobei die Ultraschallexposition an ein zentrales Nervensystem des Subjekts verabreicht wird.

10. Kombination zur Verwendung nach Anspruch 9, wobei die Bevacizumab-Formulierung 0,11 bis 25 mg/ml Bevacizumab und ein Trägersubstanz für die Injektion umfasst; wobei die mg/ml auf dem Gesamtvolumen der Bevacizumab-Formulierung basieren, wobei die Trägersubstanz für die Injektion vorzugsweise Wasser, eine Kochsalzlösung, ein Polymer, ein Emulgator, ein Tensid oder eine Kombination davon ist.

11. Kombination zur Verwendung nach Anspruch 9 oder 10, wobei das Ultraschallreaktionsmedium $1 \times 10^4$ bis $1 \times 10^{12}$ Partikel/ml Partikel umfasst; wobei die Partikel/ml auf dem Gesamtvolumen des Ultraschallreaktionsmediums basieren, wobei die Partikel vorzugsweise einen mittleren Durchmesser von 0,1 bis 10 $\mu$m aufweisen.

12. Kombination zur Verwendung nach einem der Ansprüche 9 bis 11, wobei das Ultraschallreaktionsmedium mit einer Trägersubstanz für die Injektion gemischt ist, wobei die Trägersubstanz für die Injektion vorzugsweise Wasser, eine Kochsalzlösung, ein Polymer, ein Emulgator, ein Tensid oder eine Kombination davon ist.

13. Bevacizumab zur Verwendung nach einem der Ansprüche 1 bis 9 oder die Kombination zur Verwendung nach einem der Ansprüche 10 bis 12, wobei der Gehirntumor Glioblastoma Multiforme ist.

14. Kombination zur Verwendung nach einem der Ansprüche 9 bis 13, wobei das Ultraschallsystem ein auf der medizinischen Bildgebung basierendes Führungsultraschallsystem ist.

15. Kombination zur Verwendung nach Anspruch 14, wobei die medizinische Bildgebung Neuronavigation, Ultraschalldiagnostik, optische Bildgebung, Computertomographie (CT), Kernbildgebung oder Magnetresonanztomographie (MRT) umfasst.

## Revendications

1. Bévacizumab destiné à être utilisé pour le traitement d'une tumeur cérébrale, le traitement comprenant les étapes suivantes :

   l'administration de bévacizumab à un sujet ; dans lequel ledit bévacizumab est en une quantité de 0,011 à 11 mg/kg de poids corporel pour un humain ;
   l'administration d'un milieu de réponse ultrasonore audit sujet ; et
   l'administration audit sujet d'une exposition aux ultrasons de 0,47 à 1,26 d'indice mécanique (IM),
   dans lequel ledit milieu de réponse ultrasonore est une pluralité de particules ; et dans lequel ladite exposition aux ultrasons est administrée sur un système nerveux central dudit sujet.

2. Bévacizumab destiné à être utilisé selon la revendication 1, dans lequel ladite exposition aux ultrasons est de 0,55 à 0,84 MI.

3. Bévacizumab destiné à être utilisé selon la revendication 1 ou 2, dans lequel ledit bévacizumab est en une quantité de 0,11 à 11 mg/kg de poids corporel pour un humain.

4. Bévacizumab destiné à être utilisé selon l'une quelconque des revendications 1 à 3, dans lequel ladite administration dudit bévacizumab se fait par injection intraveineuse.

5. Bévacizumab destiné à être utilisé selon l'une quelconque des revendications 1 à 4, dans lequel ladite administration pour ledit milieu de réponse ultrasonore se fait par injection intraveineuse.

6. Bévacizumab destiné à être utilisé selon l'une quelconque des revendications 1 à 5, dans lequel lesdites particules ont un diamètre moyen de 0,1 à 10 $\mu$m.

7. Bévacizumab destiné à être utilisé selon l'une quelconque des revendications 1 à 6, dans lequel ladite pluralité de particules est en une quantité de $1,9 \times 10^6$ à $1,17 \times 10^8$ particules/kg de poids corporel pour ladite administration.

8. Bévacizumab destiné à être utilisé selon l'une quelconque des revendications 1 à 7, dans lequel lesdites particules sont des microbulles.

9. Combinaison d'une formulation de bévacizumab et d'un milieu de réponse ultrasonore destinée à être utilisée pour un traitement d'une tumeur cérébrale, ladite combinaison étant comprise dans un kit, et ledit kit comprenant en outre un système à ultrasons comprenant un transducteur à ultrasons ; une administration de bévacizumab étant en une quantité de 0,011 à 11 mg/kg de poids corporel pour un humain, et ledit système à ultrasons étant réglé pour générer une exposition aux ultrasons de 0,47 à 1,26 IM,

dans laquelle ledit milieu de réponse ultrasonore est une pluralité de particules ; et dans lequel ladite exposition aux ultrasons est administrée sur un système nerveux central dudit sujet.

10. Combinaison destinée à être utilisée selon la revendication 9, dans laquelle ladite formulation de bévacizumab comprend 0,11 à 25 mg/ml de bévacizumab et un support pour injection ; ledit mg/ml étant basé sur le volume total de ladite formulation de bévacizumab, ledit support pour injection étant de préférence de l'eau, une solution saline, un polymère, un émulsifiant, un tensioactif ou une combinaison de ceux-ci.

11. Combinaison destinée à être utilisée selon la revendication 9 ou 10, dans laquelle ledit milieu de réponse ultrasonore comprend $1 \times 10^4$ à $1 \times 10^{12}$ particules/ml de particules ; ladite particules/ml étant basée sur le volume total dudit milieu de réponse ultrasonore, lesdites particules ayant de préférence un diamètre moyen de 0,1 à 10 $\mu$m.

12. Combinaison destinée à être utilisée selon l'une quelconque des revendications 9 à 11, dans laquelle ledit milieu de réponse ultrasonore est mélangé avec un support pour injection, ledit support pour injection étant de préférence de l'eau, une solution saline, un polymère, un émulsifiant, un tensioactif ou une combinaison de ceux-ci.

13. Bévacizumab destiné à être utilisé selon l'une quelconque des revendications 1 à 9, ou la combinaison destinée à être utilisée selon l'une quelconque des revendications 10 à 12, ladite tumeur cérébrale étant un glioblastome multiforme.

14. Combinaison destinée à être utilisée selon l'une quelconque des revendications 9 à 13, dans laquelle ledit système à ultrasons est un système à ultrasons de guidage basé sur l'imagerie médicale.

15. Combinaison destinée à être utilisée selon la revendication 14, dans laquelle ladite imagerie médicale comprend la neuro-navigation, l'échographie, l'imagerie optique, la tomodensitométrie (TDM), l'imagerie nucléaire ou l'imagerie par résonance magnétique (IRM).

Figure 1

Figure 2

Figure 3

EP 3 374 024 B1

Figure 4

Figure 5

Figure 6

Figure 7

Figure 8

Figure 9

Figure 10

Figure 11

Figure 12

Figure 13

Figure 14

Figure 15

# EP 3 374 024 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Strategies for enhancing antibody delivery to the brain. **RICHARD T FRANK et al.** BBA - REVIEWS ON CANCER. ELSEVIER, vol. 1816, 191-198
- **HENDRICKS BENJAMIN K et al.** NEUROSURGICAL FOCUS. *AMERICAN ASSOCIATION OF NEUROLOGICAL SURGEONS,* vol. 38 (3
- **RODRIGUEZ A et al.** *PHARMACEUTICS,* vol. 7 (3), ISSN 1999-4923, 175-187
- **IU HAO-LI et al.** *RADIOLOGY,* May 2010, vol. 255 (2), ISSN 1527-1315, 415-425
- **TSAI et al.** Ultrasonics Symposium (IUS). IEEE, 2015